# EUROPEAN PATENT APPLICATION

(11) **EP 4 471 794 A1**
(43) Date of publication of application: **04.12.2024**
(21) Application number: 24178719.1
(22) Date of filing: 29.05.2024
(51) Int. Cl.: G16H 40/20, G16H 40/40, G06N 3/08, G06N 20/00

(54) **DEVICE, DATA MANAGEMENT SYSTEM, AND METHOD FOR PROVIDING DATA**

(30) Priority: 31.05.2023 DE 102023114336
(71) Applicant: Karl Storz SE & Co. KG, 78532 Tuttlingen (DE)
(72) Inventor: Aderhold, Andrej, 78532 Tuttlingen (DE); Balashov, Alexander, 78532 Tuttlingen (DE); Khazatskiy, Igor, 78532 Tuttlingen (DE)

(57) **Abstract**

A device (100) for performing a function in a medical facility is provided, together with a data management system (1000) comprising such a device (100) as well as to a related method for providing data. The device (100) comprises:
an input interface (110) configured to receive a query (72) for data;
a functional unit (120) configured to perform a function of the device (100);
a data recorder (130) configured to record, periodically or continuously, data pertaining to the function of the device (100);
a data processor (140) configured to select, based on the query (72), a portion of the recorded data; and
an output interface (190) configured to output an output signal (73) providing the selected portion of the recorded data in response to the query (72).

## Description

### Technical field of the invention

The present invention relates to a device for performing a function in a medical facility, to a data management system, and to a computer-implemented method for providing data. It also relates to a computer program product, to a data storage medium and to a data stream. In particular, it relates to the provision or accumulation of training data for machine-learning models, in particular artificial intelligence entities.

### Background of the invention

Nowadays, more data are being collected than ever before. Many devices not only perform their original function but also produce and/or record large amounts of data, often detailing or describing said function. In principle, this data could be used to improve many devices and services, in particular when used as suitable training data for machine-learning models such as artificial intelligence entities.

However, the large amount of data on the one hand and the very diverse requirements for the properties of these data on the other hand, raise a number of issues, in particular in the context of machine learning. Often, collected data have overall low relevance for a particular project while the volume of the data may be huge. Filtering out irrelevant data in order to obtain suitable training data may be a very long process. The data may also suffer from low data reliability, for example due to a high concentration on few data sources. Furthermore, processing (for example anonymization and storage) costs for the large amounts of data may be considerable.

### Summary of the invention

The above-described problems are solved by the subject matter of the independent claims of the present invention.

Thus, according to a first aspect of the present invention, a device for performing a function in a medical facility is provided, the device comprising:
an input interface configured to receive a query for data;
a functional unit configured to perform a function of the device;
a data recorder configured to record, periodically or continuously, structured data pertaining to the function of the device;
a data processor configured to select, based on the received query, a portion of the recorded data; and
an output interface configured to output an output signal providing the selected portion of the recorded data in response to the query.

The query may last for a given amount of time, may end after a built-in condition is reached, or may stay active until it is explicitly cancelled. In this way, one or more devices may be instructed to continue to provide responses to the query. In some variants, the query will only be output if a check whether the desired data are already available in a database has come up negative.

The device may also be designated as a "front-end device" or as an edge device because typically a large number of devices will be deployed in communication with a central controller, for example the query controller introduced in the following.

The functional unit may be configured to perform any function of the device, in particular any of its main functions. For example, when the device is a medical device, a function may be a medical function such as a measurement of a body function of a patient or the like. When the device is a camera, the function may be the recording of images, optionally with sound. When the device is a medical instrument, the function may comprise cutting, gripping, sealing, and/or the like.

In single-purpose devices, the device and the functional unit may be largely (functionally) identical, for example in case of the device being a camera with, e.g., an image acquisition apparatus as its functional unit. Multi-function devices, by contrast, will comprise more than one functional unit, and/or a functional unit with more than one function. For example, the device may be a minimally invasive medical instrument, comprising an image acquisition apparatus as a first functional unit, and a tissue manipulator as a second functional unit. A third functional unit may enable applying heat to manipulated tissue, and so on.

Each device may be integrated in the same housing as its functional unit, or may be partially implemented by a set of sub-devices or sub-modules connected by a wired and/or wireless network.

The recorded data may be unstructured data such as video, image, or sound data. The recorded data may also be structured data such as text-based data, for example tables or structured reports, or predictions of a machine learning model. The recorded data may also comprise both unstructured and structured data, for example, video data (unstructured) together with meta information (structured) regarding time stamps, place of recording and the like. Recorded data comprising unstructured and structured data may also consist of unstructured data with added annotations (or: labels), such as video data together with text-based information about one or more persons or objects present in each image frame or the like. Advantageously, at least one, or even more advantageously, all of the data fields of the structured data are filled in when the recorded data are generated.

One main idea of the invention is on-demand data recording, i.e. data are identified, annotated, stored and/or transmitted within a device in response to the query. In some variants, the data may be identified, annotated, stored and/or transmitted from the device only in response to such a query, i.e. not in case there is no query. Overall, the invention has the effect of improving the relevance of data provided in response to a request for data. The invention also has the effect of a reduction of bandwidth and/or storage space needed for data provided in response to a request for data because irrelevant data is not stored and/or not transmitted.

According to a second aspect of the invention, a data management system is provided, comprising:
a plurality of devices according to any embodiment of the first aspect of the invention; and
a query controller configured to receive a request for data and to generate and transmit a query for the data according to the received request to at least one device of the plurality of devices;
wherein the query controller is further configured to receive output signals of the plurality of devices and to provide data according to the received request based on the received output signals.

The query controller may also be designated as a "central controller", or a "back-end controller" or the like, because it will act as a back-end to the plurality of devices providing the data.

Using the data management system, a user may formulate a specific request for data and input it into the query controller, and may then receive, from the query controller, the data from the plurality of the devices, or a link to the data within a database and/or the like. This simplifies for the user the process of formulating queries to a large number of devices, filtering out irrelevant data, finding out which devices produce data of the desired type, and so on.

According to a third aspect of the invention, a computer-implemented method for providing data is provided, the method comprising:
receiving, at a device for performing a function in a medical facility, a query for data (wherein preferably the device is a device according to an embodiment of the first aspect of the present invention);
performing a function of the device;
recording, regularly or continuously, data pertaining to the function of the device at the device, in particular structured data;
selecting, based on the received query, a portion of the recorded data at the device; outputting, by the device, an output signal providing the selected portion of the recorded data in response to the query.

The method according to any embodiment of the third aspect of the present invention may be implemented by any embodiment of the first aspect and/or of the second aspect of the present invention, and vice versa.

According to a fourth aspect, a computer program product comprising executable program code is provided which is configured to, when executed, perform the method of any embodiment of the method according to the third aspect of the invention.

According to a fifth aspect, a non-transitory, computer-readable data storage medium comprising executable program code is provided which is configured to, when executed, perform the method according to any embodiment of the third aspect of the invention.

According to a fifth aspect, a data stream is provided which comprises executable program code, or which is configured to generated executable program code, wherein the executable program code is configured to, when executed, perform the method according to any embodiment of the third aspect of the invention.

In some advantageous embodiments, refinements, or variants of embodiments, the recorded data comprise structured data comprising data fields indicating one or more of:
- a time, date, and/or duration of the performing of the function;
- a location of the performing of the function;
- a degree of usage or success of the function;
- at least one device, service, or functionality in the medical facility being active or inactive during the performing of the function;
- at least one device or person being present or absent during the performing of the function;
- a target of the function; and/or
- an operator of the device.

The time and date of the performing of the function may, for example, refer to time stamps of audio and/or video data, to time stamps of logged events and/or the like. The duration may be directly measured/input into a data field, or may be indicated indirectly by the difference of two time stamps.

A degree of usage may refer to a degree to which a exhaustible resource of the device is used up, a number of usages out of a fixed number of usages that the device is allowed to be used for and the like. A degree of success may refer to a degree to which a specified goal of the function of the device has been fulfilled or the like.

An example for a functionality being active during the performing of the function is, if the device is a medical instrument capable which of applying an electric current or heat to an organic tissue of a patient, when the medical instrument is performing its function (or one of its functions), for example gripping tissue, and at the same time the electric current or heat is applied. Functionality and function may be used interchangeably since a device may have multiple functions, each of which may be seen as the function of the device, and each of which may be seen as an additional functionality of the device.

A target of the function may, for example, be a patient on which the device was used to operate, for example when the device is a medical instrument.

An operator of the device may, for example, be a surgeon wielding a device being a medical instrument, a medical technical assistant programming a device and/or the like. The information about the operator may include an identity, a position, an authorization level and/or the like.

It is evident that the data fields filled out in this way may act as labels for a machine learning project. In other words, the recorded data, or at least relevant portions of it, may be automatically or pre-annotated (or: pre-labelled).

For example, if training data are needed that refer to a specific function of the device being in use and that provide insight depending on where the device has been used, the data processor may select the appropriate data based on the data corresponding data fields. This will save human assistants the time and effort to sift through endless amounts of data, cutting out all of the times when the device was not in use. The use and background of training data will be explained in more details in the following.

In some advantageous embodiments, refinements, or variants of embodiments, the device comprises an image acquisition apparatus as a functional unit. For example, the device may be a camera. A function of the device may comprise, or consist of, taking image data of a scene within a room of the medical facility, in particular an operating room, an intensive care unit, a patient room, or an anesthetic recovery room.

In some advantageous embodiments, refinements, or variants of embodiments, the device comprises a machine-learning model, MLM, in particular an artificial intelligence entity, AlE. The MLM, in particular AlE, more specifically an artificial neural network, ANN, may be configured to receive an output of the functional unit as input and to automatically generate, based thereon, at least a portion of the recorded data.

For example, the machine-learning model, MLM, may be configured to detect particular events within the recorded data. The detection, presence, duration etc. (i.e. properties) of the event may be logged in data fields of the recorded data. This may be designated as "Al-supported event detection" provided by the device. The Al-supported event detection may be based on states of the device, signals of the device, and/or machine-learning model predictions. Thus, the machine-learning model, MLM, may contribute to (or enable) the identification of conditions relevant for the query.

Machine learning may refer to algorithms and statistical models that computer systems may use to perform a specific task without using explicit instructions, instead relying on models and inference. For example, in machine learning, instead of a rule-based transformation of data, a transformation of data may be used, that is inferred from an analysis of historical and/or training data. For example, the content of images may be analyzed using a machine-learning model or using a machine-learning algorithm.

In order for the machine-learning model to analyze the content of an image, the machine-learning model may be trained using training images as input and training content information as output. By training the machine-learning model with a large number of training images and/or training sequences (e.g. words or sentences) and associated training content information (e.g. labels or annotations), the machine-learning model "learns" to recognize the content of the images, so the content of images that are not included in the training data can be recognized using the machine-learning model.

The same principle may be used for other kinds of sensor data as well: By training a machine-learning model using training sensor data and a desired output, the machine-learning model "learns" a transformation between the sensor data and the output, which can be used to provide an output based on non-training sensor data provided to the machine-learning model. The data stemming from the functional unit (e.g. sensor data, metadata and/or image data) may be preprocessed to obtain a feature vector, which is used as input to the machine-learning model.

Machine-learning models may be trained using training input data. The examples specified above use a training method called "supervised learning". In supervised learning, the machine-learning model is trained using a plurality of training samples, wherein each sample may comprise a plurality of input data values, and a plurality of desired output values, i.e. each training sample is associated with a desired output value. By specifying both training samples and desired output values, the machine-learning model "learns" which output value to provide based on an input sample that is similar to the samples provided during the training. Apart from supervised learning, semi-supervised learning may be used.

In semi-supervised learning, some of the training samples lack a corresponding desired output value. Supervised learning may be based on a supervised learning algorithm (e.g. a classification algorithm, a regression algorithm or a similarity learning algorithm. Classification algorithms may be used when the outputs are restricted to a limited set of values (categorical variables), i.e. the input is classified to one of the limited set of values. Regression algorithms may be used when the outputs may have any numerical value (within a range). Similarity learning algorithms may be similar to both classification and regression algorithms but are based on learning from examples using a similarity function that measures how similar or related two objects are.

Apart from supervised or semi-supervised learning, unsupervised learning may be used to train the machine-learning model. In unsupervised learning, (only) input data might be supplied and an unsupervised learning algorithm may be used to find structure in the input data (e.g. by grouping or clustering the input data, finding commonalities in the data). Clustering is the assignment of input data comprising a plurality of input values into subsets (clusters) so that input values within the same cluster are similar according to one or more (pre-defined) similarity criteria, while being dissimilar to input values that are included in other clusters.

Reinforcement learning is a third group of machine-learning algorithms.

In other words, reinforcement learning may be used to train the machine-learning model. In reinforcement learning, one or more software actors (called "software agents") are trained to take actions in an environment. Based on the taken actions, a reward is calculated. Reinforcement learning is based on training the one or more software agents to choose the actions such, that the cumulative reward is increased, leading to software agents that become better at the task they are given (as evidenced by increasing rewards).

Machine-learning algorithms are usually based on a machine-learning model. In other words, the term "machine-learning algorithm" may denote a set of instructions that may be used to create, train or use a machine-learning model. The term "machine-learning model" may denote a data structure and/or set of rules that represents the learned knowledge (e.g. based on the training performed by the machine-learning algorithm). In embodiments, the usage of a machine-learning algorithm may imply the usage of an underlying machine-learning model (or of a plurality of underlying machine-learning models). The usage of a machine-learning model may imply that the machine-learning model and/or the data structure/set of rules that is the machine-learning model is trained by a machine-learning algorithm.

For example, the machine-learning model may be an artificial neural network (ANN). ANNs are systems that are inspired by biological neural networks, such as can be found in a retina or a brain. ANNs comprise a plurality of interconnected nodes and a plurality of connections, so-called edges, between the nodes. There are usually three types of nodes, input nodes that receiving input values, hidden nodes that are (only) connected to other nodes, and output nodes that provide output values. Each node may represent an artificial neuron. Each edge may transmit information, from one node to another. The output of a node may be defined as a (non-linear) function of its inputs (e.g. of the sum of its inputs). The inputs of a node may be used in the function based on a "weight" of the edge or of the node that provides the input. The weight of nodes and/or of edges may be adjusted in the learning process. In other words, the training of an artificial neural network may comprise adjusting the weights of the nodes and/or edges of the artificial neural network, i.e. to achieve a desired output for a given input.

The machine-learning model, MLM, may, for example, be configured to receive at least a portion of the image data as input and to determine and classify at least one object or person therein and to generate a portion of the recorded data based thereon. Thus, one data field of the recorded data may comprise an information about any or all objects determined and classified in a particular image or video frame. In this way, a set of images, or a video stream, may be labelled (or: annotated) with the presence/absence/identity of objects in the field of view. Video data of an operating room, for example, may be labelled at each frame with information about the person(s) visible therein. Such data could be used to train a machine-learning model, MLM, to identify times of higher or lower use of the operating room or the like.

In some advantageous embodiments, refinements, or variants of embodiments, the input interface is configured to receive a query for training data for machine learning. The output signal may comprise training data with entries of at least one data field of the recorded data being used as label(s). In this way, any or all of the deployed devices may contribute in generating specifically required training data for machine learning.

In some advantageous embodiments, refinements, or variants of embodiments, the device comprises, or consists of, a medical instrument, in particular an endoscope.

In some advantageous embodiments, refinements, or variants of embodiments, the data processor is further configured to post-process and/or anonymize the recorded data in accordance with requirements of the query. In this way, legal or other requirements such as the GDPR of the European Union can be easily observed. The data processor may, additionally or alternatively, also be configured to post-process and/or anonymize the recorded data in accordance with requirements of a data policy of the medical facility.

In some advantageous embodiments, refinements, or variants of embodiments, the query controller of the data management system is implemented by a cloud computing device, such as a cloud computing platform, e.g., implemented by one or more remote servers. The cloud computing device (specifically the query controller, or a dedicated testing module or the like) may be further configured to perform a data verification and/or a compliance check on the received output signals. It may be configured such that only data that pass all checks performed on them are provided in response to the received request.

For example, the query controller or testing module may test whether all personal or non-anonymous information has been successfully removed from each output signal, and may only provide such data in response to the request for data that pass this test. This ensures that GDPR or other regulations are followed without the need for a user to manually test the output signals individually, and/or without testing having to be performed by each device individually, although that may also be possible (e.g. implemented by the data processor).

In some advantageous embodiments, refinements, or variants of embodiments, the query controller is configured to output a notification signal indicating availability of data according to the received request for data. In a simple case, the notification signal may inform the user who has input the request for data whether the requested data are available immediately (e.g. when the same request has been previously made and the corresponding data has already been provided in response thereto before), or whether the requested data must first be produced, or collected. The notification signal may also comprise a link with which the user may access, e.g. download, the provided data.

It may take a while for enough output signals to be received by the query controller in response to a request for data. The notification signal may indicate to a user that has formulated the request how far the process of receiving a sufficient number (e.g. a pre-set number or a number given by the user with the request) has progressed, how much time the fulfillment of the request may (probably) take, or simply that enough/sufficient/suitable data have now been collected and can now be provided to the user in response to the request. The notification signal may be output to the user in any known way, e.g. via an email, an app notification and/or the like.

In some advantageous embodiments, refinements, or variants of embodiments, the method according to the third aspect of the present invention further comprises:
generating the query for the data;
transmitting the query to at least one device of a plurality of devices;
receiving at least one output signal in response to the query; and
generating a data structure in response to the query based on the received output signals.

In some advantageous embodiments, refinements, or variants of embodiments, the generated data structure is a training data set for machine learning, wherein entries of at least one data field of the structured data are used as labels.

Additional advantageous features and effects will become apparent in connection with the dependent claims as well as in connection with the detailed description of the invention as well as the corresponding drawings.

Further applicability of the present invention will become apparent from the following figures, detailed description and claims. However, it should be understood that the detailed description and specific examples, while indicating preferred embodiments of the invention, are given by way of illustration only, since various changes and modifications within the spirit and scope of the invention will become apparent to those skilled in the art.

### Brief description of the figures

Aspects of the present disclosure will be better understood with reference to the following figures. The components in the drawings are not necessarily to scale, emphasis being placed instead upon clearly illustrating the principles of the present disclosure. Parts in the different figures that correspond to the same elements have been indicated with the same reference numerals in the figures, in which:
- Fig. 1: shows a schematic block diagram illustrating devices according to an embodiment of the first aspect of the present invention as well as a data management system according to an embodiment of the second aspect of the present invention;
- Fig. 2: shows a schematic block diagram of a device according to another embodiment of the present invention;
- Fig. 3: is a flow diagram illustrating a computer-implemented method for providing data according to yet another embodiment of the present invention;
- Fig. 4: is a block diagram illustrating a computer program according to still another embodiment of the present invention; and
- Fig. 5: is a block diagram illustrating a data storage medium according to still a further embodiment of the present invention.

The figures are not drawn to scale, and the components are shown in generalized or schematic form in the interest of clarity and conciseness. In some instances, well-known structures and devices are shown in block diagram form in order to avoid obscuring the concepts of the present invention.

### Detailed description of the figures

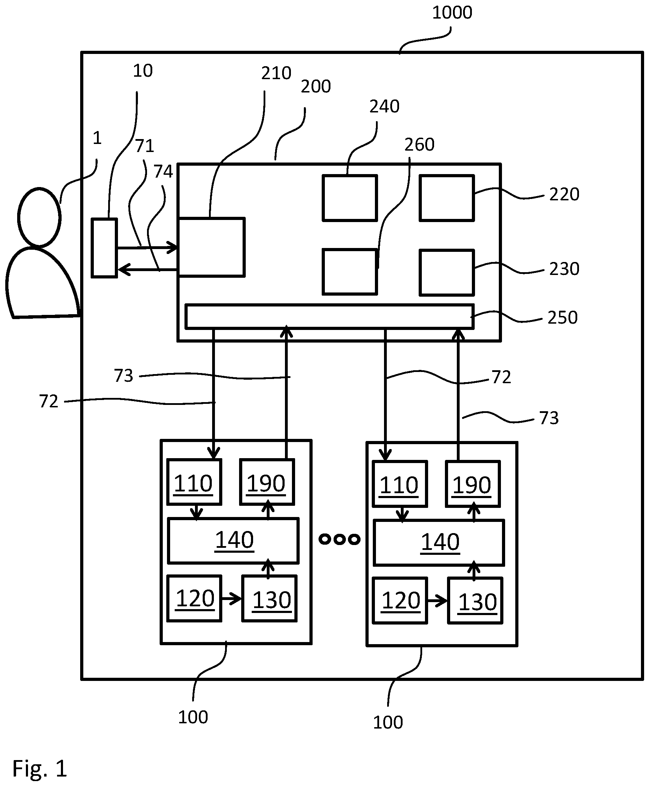

Fig. 1 shows a schematic block diagram illustrating devices 100 according to an embodiment of the first aspect of the present invention as well as a data management system 1000 according to an embodiment of the second aspect of the present invention. Thus, Fig. 1 illustrates devices 100 for performing functions in a medical facility.

Fig. 1 illustrates the devices 100 as part of the data management system 1000 but it shall be understood that the devices 100 are also independent objects of the present invention. Although specifically two devices 100 are shown in Fig. 1 it shall be understood that the system 1000 may comprise any number of devices 100, in particular more than fifty or more than one hundred devices 100.

Apart from the devices 100, the data management system 1000 comprises a query controller 250 configured to receive a request 71 for data and to generate and transmit a query 72 for the data according to the received request 71 to at least one device 100 of the plurality of devices 100, preferably to a plurality or to all of the devices 100. The query controller 250 may be implemented by a cloud computing device 200 as one part of the data management system 1000. The query controller 250 is part of a network that also includes the devices 100, wherein the network itself may or may not be part of the data management system 1000. The network may be the Internet, the Internet of Things, an Ethernet network and/or the like.

The query controller 250 may be configured to transmit the query 72 to all devices 100 or to a selection of the devices 100. The selection may be random, based on properties of the network (e.g. latency, bandwidth and so on in connection with particular devices 100), or based on properties of the devices 100 themselves.

For example, only such devices 100 may be selected which are known to be able to produce the data to which the query 72 pertains. If, for instance, labelled video data are requested by the query 72, then only such devices 100 may be selected to receive the query 72 which are known to produce video data in the first place, or known to produce specifically video data of a kind specially required by the query 72. A database 240 regarding the capabilities of the devices 100 to fulfill different types of requests may be stored within the query controller 250 or, as shown in Fig. 1, within the cloud computing device 200.

The database 240 may continue to be updated. For example, the query controller 250 may be configured to send out the queries 72 to all devices 100 for which it is not known that they are incapable of fulfilling a particular query 72. These may include new devices 100 which are connected to the query controller 250 over the same network as the others but for which the query controller 250 does not yet have any information. The output signals 73 by the devices 100 may include information indicating to the query controller 250 the present and/or absent capabilities of each particular device 100 so that the query controller 250 may update the database 240 based on this information. Another option is that each device 100 that is newly connected to the network in which the query controller 250 is connected, transmits (either by itself or in response to a signal by the query controller 250) its capabilities to the query controller 250 for storage in the database 240.

For example, the request 71 for data may be generated by a user 1 using a local device 10 such as a smartphone, a tablet, a PC, a workstation, a laptop, a notebook or the like. The local device 10 may be part of the data management system 1000 as well. Specifically, the local device 10 may be a terminal of a machine learning engineer (data scientist). The request may be formulated using a graphical user interface, GUI 210, provided by the cloud computing device 200. The request 71 may contain, for example, specific requirements for number of data, content of data, and labels for data for a machine-learning project. The GUI may be implemented by the cloud computing device 200 as shown in Fig. 1, for example as a browser-based web service, and/or by an app running on the local device 10 of the user 1.

The query controller 250 is further configured to receive output signals 73 of the plurality of devices 100 and to provide data 74 according to the received request 71 based on the received output signals 73. The data 74 may be provided to the user 1 using the graphical user interface, GUI 210. The GUI 210 may provide the user 1 with analysis functions regarding the provided data 74 such as a data browser, statistics overview or the like. Thus, the user 1 may be enabled to determine whether the request 71 was correctly formulated and/or correctly interpreted or responded to, may re-formulate the request 71, input another request 71 and so on. The GUI 210 may also provide the user with the ability to search through available data and historical data queries.

The data management system 1000 may further comprise, or implement, a testing module 220 configured to perform one or more tests on the received output signals 73. These tests may include data verification, compliance tests and/or the like, as has been described in the foregoing. The testing module 220 may be implemented within the query controller 250, as part of the cloud computing device 200, operatively linked to the query controller 250 or in any other suitable way.

Although here, in the foregoing and in the following, some functions are described as being performed by modules, it shall be understood that this does not necessarily mean that such modules are provided as entities separate from one another. In cases where one or more modules are provided as software, the modules may be implemented by program code sections or program code snippets which may be distinct from one another but which may also be interwoven.

Similarly, in case where one or more modules are provided as hardware, the functions of one or more modules may be provided by one and the same hardware component, or the functions of one module or the functions of several modules may be distributed over several hardware components which need not necessarily correspond to the modules one-to-one. Thus, any apparatus, system, method and so on which exhibits all of the features and functions ascribed to a specific module shall be understood to comprise, or implement, said module.

In particular, it is a possibility that all modules are implemented by program code executed by a computing device, e.g. a server or a cloud computing platform.

The testing module 220 may, additionally or alternatively, also have the purpose of performing a data match check, i.e. of testing whether the data received in the output signals 73 actually matches the request 71 for data and/or the query 72. Data not matching the specified criteria in either request 71 or query 72, and/or not fulfilling other tests (such as sufficient data protection tests or the like) may be dropped and thus not become part of the provided data 74.

The provided data 74 may be stored in a database 260 (or: cloud data lake), for example in case of further identical or similar requests 71. The database 260 may be part of the query controller 250 or, as shown in Fig. 1, be implemented separately by the cloud computing device 200. When such data are stored in the database 260, the query controller 250 may, when a new request 71 is received, first check whether the request 71 can already be fulfilled using the data stored in the database 260. It may generate and transmit the query 72 only in case that the data (if any) already stored in the database 260 is insufficient.

For the sake of easier understanding, two separate databases 240, 260 are shown in Fig. 1, whereas it shall be understood that separate database instances may be provided for the storage of different types of data, or a single database may be provided for all data storage needs of the query controller 250 and/or the cloud computing device 200. In particular, the database 240 and/or the database 260 may be integrated into each other and/or may be distributed over several pieces of hardware equipment.

A computing device (e.g., the cloud computing device 200, the query controller 250 and/or the data processor to be described in the following) may be realized as any device, or any means, for computing, in particular for executing a software, an app, or an algorithm. For example, the computing device may comprise at least one processing unit such as at least one central processing unit, CPU, and/or at least one graphics processing unit, GPU, and/or at least one field-programmable gate array, FPGA, and/or at least one application-specific integrated circuit, ASIC and/or any combination of the foregoing. The computing device may further comprise a working memory operatively connected to the at least one processing unit and/or a non-transitory memory operatively connected to the at least one processing unit and/or the working memory.

The cloud computing device 200 may also comprise a training module 230 configured to train a machine-learning model, MLM, such as an artificial intelligence entity, AlE, specifically an artificial neural network, ANN, based on the provided data 74. The functions of the training module 230 may be accessible to the user 1 via the graphical user interface, GUI 210, as well. In other words, the GUI 210 may also be configured to design an architecture of a machine-learning model, MLM specifically of an artificial neural network, ANN, and to formulate a request 71 for data 74 as suitable training data.

Each device 100 comprises an input interface 110 configured to receive the query 72 for data. As has been described in the foregoing, although the function of the devices 100 is in the context of Fig. 1 described together with the data management system 1000, the cloud computing device 200 and the query controller 250, its functions may also be implemented independently of any of these elements. The query 72 for data may thus also stem from a different source, for example directly from a user 1.

It should also be understood that, although both exemplary devices 100 in Fig. 1 are depicted in the same abstract manner, any or all of the devices 100 may be completely different in their functions, setup, properties, hardware, software, and so on.

Each device 100 further comprises a functional unit 120 configured to perform a function of the device 100, or several functional units 120, each connected to one or more functions of the device 100. For example, one device 100 may be a camera, with the functional unit 120 being, or comprising, the image acquisition apparatus (comprising for example an image recording chip, a control circuit etc.). Another device 100 may be a medical instrument such as an endoscope.

Each device 100 further comprises a data recorder 130 configured to record, periodically or continuously, data pertaining to the function of the device 100, in particular by measuring, receiving, copying or otherwise obtaining data from one or more functional unit 120. The data recorder 130 may request the data periodically, or the functional unit(s) 120 may be configured to continuously transmit the data to the data recorder 130, or any other system for obtaining data known to the skilled person may be employed. It shall be understood that this may also be handled differently in each device 100, in particular depending on any function(s) of the functional unit(s) 120.

Similarly, each data recorder 130 may be configured to store all data it obtains, to store only a portion, and so on. Each data recorder 130 may also be configured to store data based on the source of the data, for example based on the type of function unit 120 from which the data stems. For example, data from a pump may be stored continuously, while data from a light source may be stored periodically or not at all, or vice versa.

As has been described in the foregoing, it is advantageous if the recorded data at least comprise structured data (such as data files consisting of text and/or numbers), either exclusively or in addition to unstructured data (such as video and/or audio files), although also cases with only unstructured data are possible.

The structured data may comprise, or include, data fields indicating one or more of:
- a time, date, and/or duration of the performing of the function;
- a location of the performing of the function;
- a degree of usage or success of the function;
- at least one device, service, or functionality in the medical facility being active or inactive during the performing of the function;
- at least one device or person being present or absent during the performing of the function;
- a target of the function; and/or
- an operator of the device 100.

Each device 100 further comprises a data processor 140 configured to select, based on the received query 72, a portion of the recorded data. The selection may in particular be based on any or all data fields of the structured data of the recorded data, since these are in general easier to process. The selection may be temporal (only a certain time period), spatial (only data relating to, or gathered in, a particular location), logical (only data fulfilling certain logical criteria), personal (only data relating to, or generated by, particular persons) and/or the like. The data processor 140 and the data recorder 130 may be integrated into one another, for example when the device 100 is designed such that only data selected by the data processor 140 are to be stored.

The data processor 140 may also be configured to post-process and/or anonymize the recorded data in accordance with requirements of the query.

Each device 100 also comprises an output interface 190 configured to output an output signal 73 providing the selected portion of the recorded data in response to the query 72.

The query controller 250 is configured to collect the output signals 73 and to generate from them the data 74 to be provided. The query controller 250 may also cause a notification signal to be sent to the user 1, for example using the graphical user interface 210, informing them that the data 74 according to the request 71 has been provided. The notification may include a link to the data stored in the database 260 of the cloud computing device 200. Similarly, an ongoing request 71/query 72 may result in new batches of data being stored in the database 240 such that a notification signal may also inform the user 1 of such a new batch having been provided.

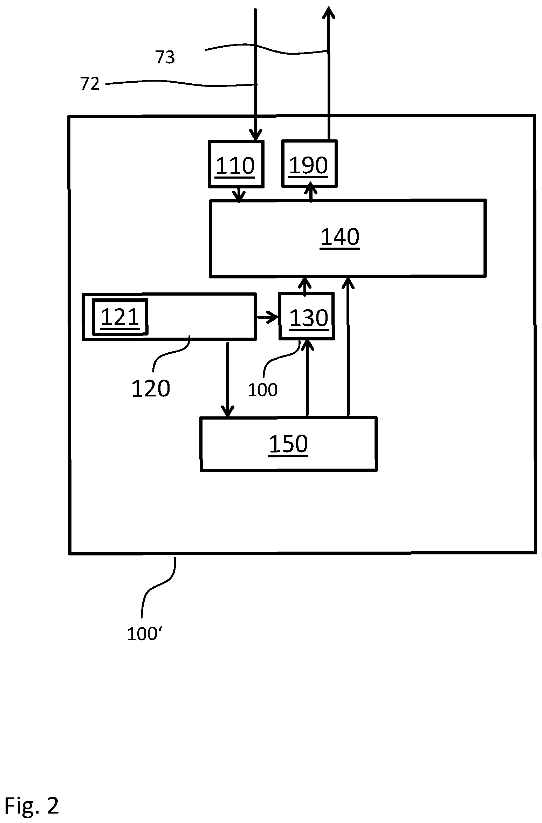

Fig. 2 shows a block diagram illustrating a camera 100' as one type of a device 100 according to an embodiment of the present invention, in particular of its first aspect.

The function of the camera 100' may comprise, or consist of, taking image data of a scene within a room of the medical facility, in particular an operating room, an intensive care unit, a patient room, an anesthetic recovery room, or the like. The purpose of the function may be to monitor the presence of any or of specific people, to record events for later analysis or training and the like. The medical facility in question may thus be a hospital, a medical research institute, a private medical practice, and so on.

In Fig. 2, the functional unit 120 of the camera 100' comprises, or consists of, an image acquisition apparatus 121, comprising, for example, an image acquisition chip, a controlling circuit and so on.

The camera 100' may also comprise a machine-learning model 150 (or a machine-learning algorithm), for example a trained artificial neural network, ANN, either as part of the data processor 140 or implemented separately, as shown in Fig. 2.

The machine-learning model 150 may be trained and configured to detect events and/or objects within the images acquired by the image acquisition apparatus 121 and/or to make predictions. The storing of data by the data recorder 130 and/or the selecting of data by the data processor 140 may be based on the detected events and/or objects and/or predictions. In other words, the data recording by the data recorder 130 may be triggered by Al-supported event detection, and/or the data relevant for the query may be identified by the data processor 140 using Al-supported event detection.

For example, the query 72 for data may refer to only image or video data containing people. The machine-learning model 150 may thus identify or annotate all image data generated by the image acquisition apparatus 121 which comprise people, and the data recorder 130 and/or the data processor 140 may then make the corresponding selection(s). In this way, the output signals 73 will not comprise any data that does not comprise people, thus significantly reducing the necessary bandwidth and storage capacity.

In the examples of Fig. 1 and Fig. 2, the devices 100, 100' have been shown as integrated devices, preferably integrated within a housing. Examples for such devices 100, 100' include a light source (functional unit e.g. light engine), an insufflator (functional unit e.g. compressor), a control terminal (functional unit e.g. CPU), a camera control unit (CCU) and/or the like.

However, in other variants, the device 100, 100' may be configured in a distributed manner, e.g. as a set of sub-devices connected via a wired or wireless network. For example, the data recorder 130 and/or the data processor 140 and/or the machine learning model 150 could be arranged in the same housing as the functional unit 120, or it/they could be implemented in one or more external networked device(s).

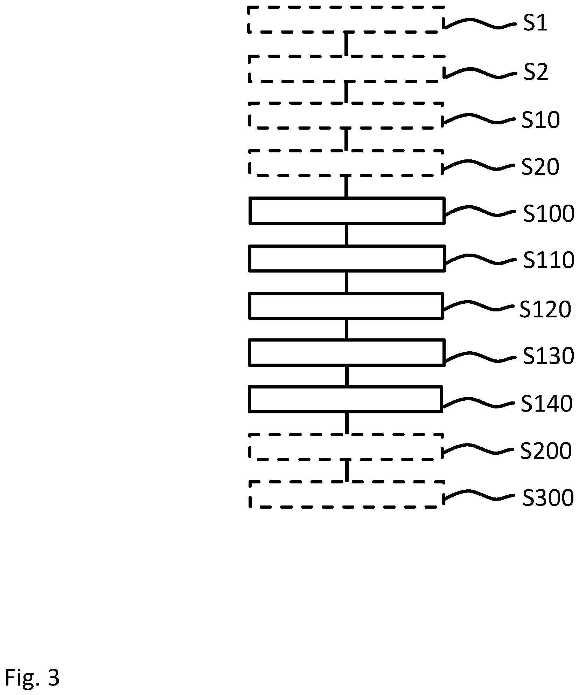

Fig. 3 is a flow diagram illustrating a computer-implemented method for providing data according to the third aspect of the present invention. The method may be performed using any embodiment of the devices according to the first aspect of the present invention and/or any embodiment of the data management system according to the second aspect of the present invention, in particular any of the devices 100 or the data management system 1000 described in the foregoing with respect to Fig. 1 and/or Fig. 2. Thus, any variant, option, or refinement described for any of these devices or data management systems may be equally applied to the method of Fig.3, and vice versa.

In a step S100, a query 72 for data is received at a device 100 for performing a function in a medical facility, for example as has been described in connection with Fig. 1.

In a step S110, a function of the device 100 is performed, in particular by a functional unit 120 of the device 100 as has been described in the foregoing.

In a step S120, data pertaining to the function of the device 100 are recorded regularly or continuously at the device 100, in particular as has been described in the foregoing with respect to the data recorders 130 of the devices 100.

In a step S130, a portion of the recorded data is selected at the device 100 based on the received query 72, for example as has been described with respect to the data processor 140 in the foregoing.

In a step S140, an output signal 73 is output by the device 100, which provides the selected portion of the recorded data in response to the query 72.

The method may optionally comprise further steps related to the generating of the query 72 and/or its further processing.

In a step S1, a user 1 may generate a request 71 for data.

In a step S2, it may be checked whether the request 71 can already be fulfilled, for example because the database 260 already contains the required data. If this is the case, these data may be provided to the user 1 in response to the request 71. If this is not the case, the remaining steps of the method are performed (i.e. the on-demand data generation or collection).

In a step S10, the query 72 for the data may be generated, for example as has been described in the foregoing by the query controller 250 in response to a request 71 for data formulated (or: generated) by a user 1.

In a step S20, the query 72 is submitted to at least one device 100 of a plurality of devices 100, in particular by a query controller 250 as described in the foregoing. The selection to which devices 100 the query 72 is submitted may depend on the specific implementation of the method and/or on the query 72 at hand. For example, the selection may be random, may be based on properties of a network connecting the devices 100 and the query controller 250, or it may be based on properties of the devices 100, e.g., as has been described in the foregoing.

In a step S200, at least one output signal 73 is received in response to the query 72, in particular at the query controller 250 as has been described in the foregoing.

In an optional step S300, a data structure is generated in response to the query 72 based on the received output signals 73. For example, a set of labelled training data may be stored, or a machine-learning model may be trained using the training module 230 such that the data structure being generated is a trained machine-learning model (e.g. a trained artificial intelligence entity, AlE, such as a trained artificial neural network, ANN). A notification signal may be output to the user 1, and/or the requested data 74 may be provided to the user 1.

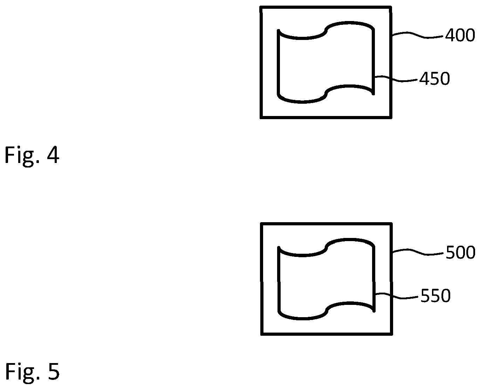

Fig. 4 shows a schematic block diagram illustrating a computer program product 400 according to an embodiment of the fourth aspect of the present invention. The computer program product 400 comprises executable program code 450 configured to, when executed, perform the method according to any embodiment of the third aspect of the present invention, in particular as has been described with respect to the preceding figures.

Fig. 5 shows a schematic block diagram illustrating a non-transitory computer-readable data storage medium 500 according to an embodiment of the fifth aspect of the present invention. The data storage medium 500 comprises executable program code 550 configured to, when executed, perform the method according to any embodiment of the third aspect of the present invention, in particular as has been described with respect to the preceding figures.

The non-transient computer-readable data storage medium may comprise, or consist of, any type of computer memory, in particular semiconductor memory such as a solid-state memory. The data storage medium may also comprise, or consist of, a CD, a DVD, a Blu-Ray-Disc, an USB memory stick or the like.

The previous description of the disclosed embodiments are merely examples of possible implementations, which are provided to enable any person skilled in the art to make or use the present invention. Various variations and modifications of these embodiments will be readily apparent to those skilled in the art, and the generic principles defined herein may be applied to other embodiments without departing from the spirit or scope of the present disclosure.

Thus, the present invention is not intended to be limited to the embodiments shown herein but it is to be accorded the widest scope consistent with the principles and novel features disclosed herein. Therefore, the present invention is not to be limited except in accordance with the following claims.

### List of Reference Signs

- 10: local device
- 71: request for data
- 72: query for data
- 73: output signal
- 74: provided data
- 100: device
- 100': camera
- 110: input interface
- 120: functional unit
- 130: data recorder
- 140: data processor
- 150: machine learning model
- 190: output interface
- 200: cloud computing device
- 210: graphical user interface
- 220: testing module
- 230: training module
- 240: database
- 250: query controller
- 260: database
- 400: computer program product
- 450: program code
- 500: data storage medium
- 550: program code
- 1000: data management system
- S1..S300: method steps

## Claims

1. A device (100; 100') for performing a function in a medical facility, comprising:
an input interface (110) configured to receive a query (72) for data;
a functional unit (120) configured to perform a function of the device (100; 100');
a data recorder (130) configured to record, periodically or continuously, data pertaining to the function of the device (100; 100');
a data processor (140) configured to select, based on the query (72), a portion of the recorded data; and
an output interface (190) configured to output an output signal (73) providing the selected portion of the recorded data in response to the query (72).

2. The device (100; 100') of claim 1, wherein the recorded data comprise structured data comprising data fields indicating one or more of:
- a time, date, and/or duration of the performing of the function;
- a location of the performing of the function;
- a degree of usage or success of the function;
- at least one device, service, or functionality in the medical facility being active or inactive during the performing of the function;
- at least one device or person being present or absent during the performing of the function;
- a target of the function; and/or
- an operator of the device (100; 100').

3. The device (100') of claim 1 or claim 2, comprising an image acquisition apparatus (121) as a functional unit (120), wherein a function of the device (100') comprises taking image data of a scene within a room of the medical facility, in particular an operating room, an intensive care unit, a patient room, or an anesthetic recovery room using the image acquisition apparatus (121).

4. The device (100) of any of claims 1 to 3, further comprising
a machine-learning model, MLM (150), in particular an artificial intelligence entity, AlE, configured to receive an output of the functional unit (120) as input and to automatically generate, based thereon, at least a portion of the recorded data.

5. The device (100') of claim 3 and claim 4, wherein the MLA (150) is configured to receive at least a portion of the image data as input and to determine and classify at least one object therein and to generate a portion of the recorded data based thereon.

6. The device (100; 100') of any of claims 1 to 5,
wherein the input interface (110) is configured to receive a query for training data for machine learning, and wherein the output signal (73) comprises training data with entries of at least one data field of the recorded data being used as labels.

7. The device (100; 100') of any of claims 1 to 6, wherein the device (100; 100') comprises or consists of a medical instrument, in particular an endoscope.

8. The device (100; 100') of any of claims 1 to 7, wherein the data processor (140) is further configured to post-process and/or anonymize the recorded data in accordance with requirements of the query and/or requirements of a data policy of the medical facility.

9. A data management system (1000), comprising:
a plurality of devices (100; 100') according to any of claims 1 to 8; and
a query controller (250) configured to receive a request for data (71) and to generate and transmit a query (72) for the data according to the received request (71) to at least one device (100; 100') of the plurality of devices (100; 100');
wherein the query controller (250) is further configured to receive output signals (73) of the plurality of devices and to provide data (74) according to the received request (71) based on the received output signals (73).

10. The data management system (1000) of claim 9,
wherein the query controller (250) is implemented by a cloud computing device (200), and wherein the cloud computing device (200) is further configured to perform a data verification and/or a compliance check on the received output signals (73).

11. The data management system (1000) of claim 10,
wherein the query controller (250) is configured to output a notification signal indicating availability of data according to the received request (71) for data.

12. A computer-implemented method for providing data, comprising:
receiving (S100), at a device (100; 100') for performing a function in a medical facility, a query (72) for data;
performing (S110) a function of the device (100; 100');
recording (S120), regularly or continuously, data pertaining to the function of the device (100) at the device (100; 100');
selecting (S130), based on the received query (71), a portion of the recorded data at the device (100; 100');
outputting (S140), by the device (100; 100'), an output signal (73) providing the selected portion of the recorded data in response to the query (72).

13. The method of claim 12,
further comprising:
generating (S10) the query (72) for the data;
transmitting (S20) the query (72) to at least one device (100) of a plurality of devices (100; 100');
receiving (S200) at least one output signal in response to the query; and
generating (S300) a data structure in response to the query (72) based on the received output signals (73).

14. The method of claim 12 or claim 13,
wherein the generated data structure is a training data set for machine learning, wherein entries of at least one data field of the structured data are used as labels.

15. A computer program product (400) comprising executable program code (450) configured to, when executed, perform the method of any of claims 12 to 14.

16. A non-transitory, computer-readable data storage medium (500) comprising executable program code (550) configured to, when executed, perform the method of any of claims 12 to 14.
